# EUROPEAN PATENT SPECIFICATION

(11) **EP 3 527 174 B1**
(45) Date of publication and mention of the grant of the patent: **13.05.2020**
(21) Application number: 18156990.6
(22) Date of filing: 15.02.2018
(51) Int. Cl.: A61F 2/30, A61F 2/42, A61F 2/38

(54) **PROSTHESIS INCLUDING BALL AND SOCKET ARRANGEMENT**
PROTHESE MIT KUGELVERBINDUNGSANORDNUNG
PROTHÈSE COMPRENANT UN AGENCEMENT DE JOINT À ROTULE

(43) Date of publication of application: 21.08.2019
(73) Proprietor: Aptis Medical, LLC, Glenview, KY 40025 (US)
(72) Inventor: SCHEKER, Luis Roman, Glenview, KY Kentucky 40025 (US)
(74) Representative: J A Kemp LLP

(56) References cited:
- US-A1- 2013 197 655
- US-B1- 8 052 757
- US-B2- 8 048 162

## Description

### BACKGROUND

The present invention relates to a prosthesis that includes a ball-and-socket arrangement. In particular, it includes a socket that includes a base and cap to capture a "ball", wherein the base and the cap of the socket are releasably secured by a fastener.

### SUMMARY

The prosthesis includes a first body, including a ball, and a second body, including a base and a cap which secures to the base. The base and cap each have an inner surface and an outer surface, and the inner surfaces of the base and cap together define a socket which is sized and shaped to receive the ball and support the ball for rotation relative to the second body. One of the inner and outer surfaces of the base defines a first groove, and the cap has a first leg, which is received in the first groove. The first leg defines a first leg opening having an axis extending in a cross-wise direction that is cross-wise to the direction in which the leg projects into the groove, and the base defines a base opening which is aligned with the first leg opening when the first leg is received in the first groove. A pin is received in the first leg opening and the base opening and extends in the cross-wise direction to secure the cap and base members together so that when forces are applied to attempt to slide the leg back out of the groove, those forces put the pin in shear, which provides a very secure connection.

### BRIEF DESCRIPTION OF THE DRAWINGS

Figure 1 is a front view of a prior art wrist prosthesis installed on a human skeleton;
Figure 2 is an exploded perspective view of the prior art wrist prosthesis of Figure 1;
Figure 3 is an exploded view of an embodiment of a wrist prosthesis, similar to that of Figure 2, but made in accordance with an embodiment of the present invention;
Figure 4 is a perspective view of the pin fastener of the prosthesis of Figure 3;
Figure 5 is a perspective view of the cap of the prosthesis of Figure 3;
Figure 6 is a perspective view of the base of the prosthesis of Figure 3, but somewhat modified, with the cup 70 removed and a pin 68 added;
Figure 7 is a side view of the prosthesis assembly incorporating the fastener, the cap of Figures 3 and 5, and the base of Figure 6;
Figure 7A is a section view along line 7A-7A of Figure 7;
Figure 7B is a section view, similar to that of Figure 7A, but for an alternate embodiment;
Figure 8 is a perspective view of a prior art elbow prosthesis installed on a human skeleton;
Figure 9 is an exploded view of an elbow prosthesis made in accordance with an embodiment of the present invention to replace the elbow prosthesis of Figure 8;
Figure 10 is a skeletal, front view showing a thumb prosthesis installed on a human hand; and
Figure 11 is an exploded view of the thumb prosthesis of Figure 10.

### DESCRIPTION

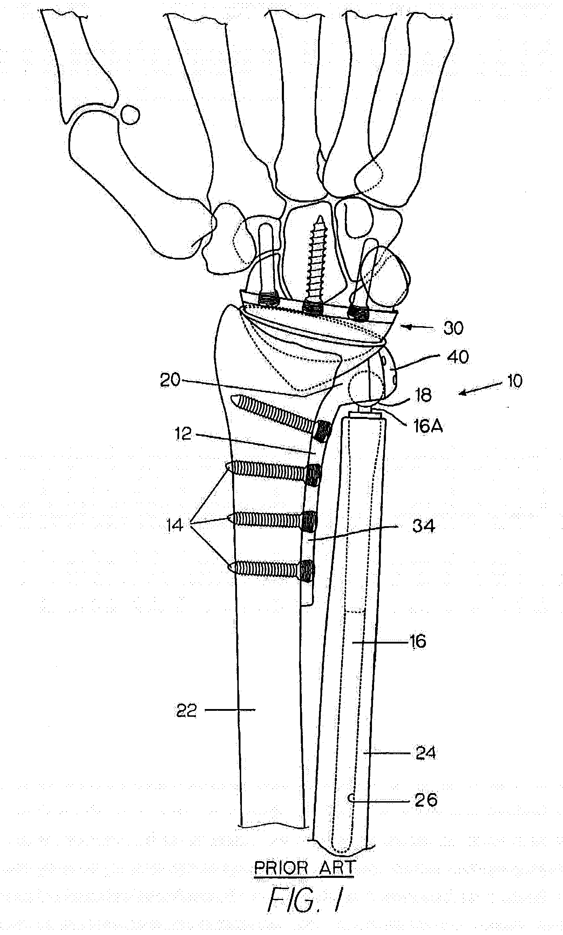

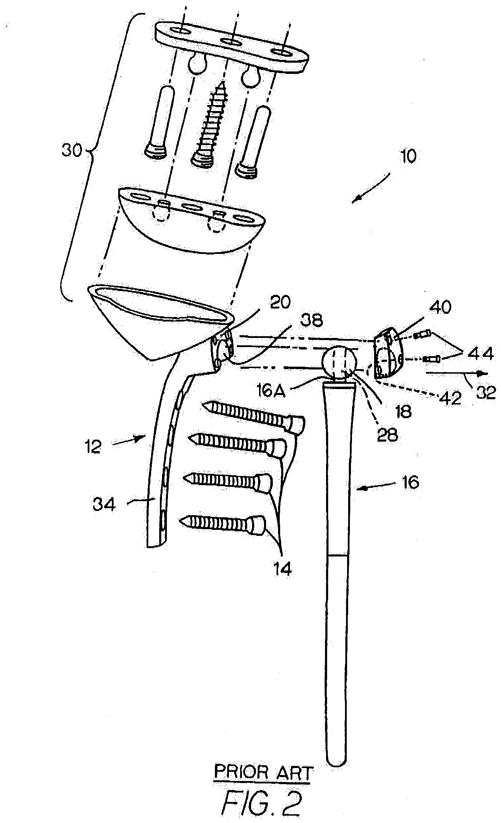

Figures 1 and 2 show a wrist prosthesis 10, as described in U. S. Patent No. 8,052,757, "Combined total wrist and total distal radioulnar joint prosthesis", issued November 8, 2011. The wrist prosthesis 10 includes a radial brace body 12, which is secured to the radius bone 22 with a plurality of screws 14. Also included is an ulnar brace body 16, which is secured to the ulna bone 24, typically via a press fit into the medullary cavity 26 of the ulna 24. In addition to (or instead of) the press fit, the brace body 16 may be cemented, adhered, or secured by other means to the ulna 24.

The ulnar brace body 16 is essentially a shaft, symmetrical about a central axis. A spherical ball 18 is mounted onto the shaft 16 at one end. The ball 18 has a bore 28 (See Figure 2) along its diameter which receives a reduced cross-section end portion 16A of the shaft 16. The ball 18 is free to pivot about the axis of the shaft 16 and to translate up and down along the central axis relative to the shaft 16.

As shown best in Figure 2, the radial brace body 12 includes a base portion 20, which has an inner surface 38 that defines a partial spherical cavity or socket. The cap 40 has an inner surface 42, which also defines a partial spherical cavity or socket. When the cap 40 is secured to the base 20 by means of screws 44, the inner surfaces 38, 42 together define a spherical socket which is sized and shaped to receive the ball 18 and to support the ball 18 for rotation. The spherical ball 18 of the ulnar brace body 16 is trapped in the partial spherical cavity formed by the base portion 20 and cap 40 and is free to swivel within and bear against the surface of that cavity, in order to support the radius 22 relative to the ulna 24 for pronation and supination of the forearm and for the bearing of weight. The bearing surface 38 is nearly in the position of the sigmoid notch of the original radius, and the ball 18 is nearly in the position of the ulna head of the original ulna, so the joint provides the same relative positions of the radius and ulna throughout the entire pronation and supination of the forearm as would have been provided by the original intact joint. This means both that the axes of the radius 22 and ulna 24 are in the same relative positions and that the longitudinal position of the joint along the axes of both bones is in the same relative position as it would have been in the original joint. The socket has a smooth, nearly continuous surface to provide for smooth rotation of the ball in the socket.

The socket ensures that the ball 18 of the ulnar brace 16 will remain engaged with the radial brace 12 so it can bear against the bearing surfaces 38, 42 to provide the desired support. The socket essentially replaces the function of the ligaments that originally held the radius in position relative to the ulna head.

The base 20 also has a mounting leg 34, projecting away from the socket, for mounting the socket to the radius bone 22 via the screws 14.

When the ball 18 bears against the bearing surface 42, it acts to push the cap 40 away from the base 20, in the axial direction of the screws 44, as shown by the arrow 32 (See Figure 2), which places the screws 44 in tension and depends upon the engagement of the screw threads to hold the assembly together. Repeated thrusts of the ball 18 on the bearing surface 42 due to loading and unloading of the wrist may result in failure of the screws 44 or in undesirable unthreading of these screws 44. Of course, a solution to this problem could be to glue, or otherwise permanently secure, the screws 44 to the cap 40 and/or to the base 20 so they will not inadvertently come unscrewed. However, this is undesirable, because it would hamper the ability of a surgeon to remove the cap 40 from the base 20 at some later time in order to replace a worn ball 18.

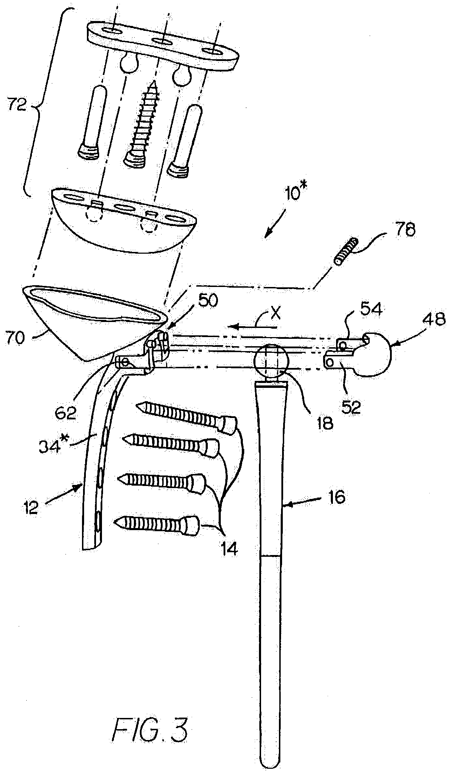

Figure 3 shows a wrist prosthesis 10* similar to what is shown in Figure 2, but with a base and cap having a more secure assembly arrangement. This arrangement does not rely upon the engagement of the threads to hold the assembly together, because the force on the fastener pin 78 is not **along** the pin's axis, putting the pin in tension, as was the case in Figure 2, but rather the force on the fastener pin 78 is **across** the pin's axis, putting the fastener pin 78 in shear, which makes for a much stronger, more secure connection.

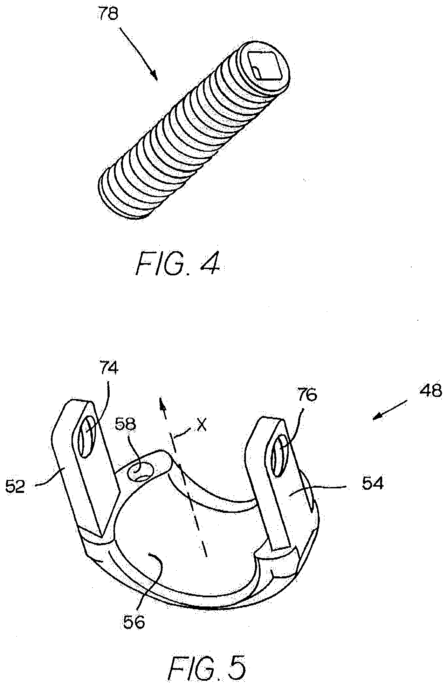

Referring to Figure 5, the cap 48 has an inner surface 56 that defines a partial sphere. First and second legs 52, 54, which are parallel and opposite to each other, project in a first direction "X". The legs 52, 54 of the cap 48 define internally threaded, coaxial, through openings 74, 76, respectively, which receive and mate with the threads on the fastener pin 78 (See Figures 3 and 4) as described in more detail later. The fastener pin 78 may be inserted either from the side of the first leg 52 or from the side of the second leg 54, as desired.

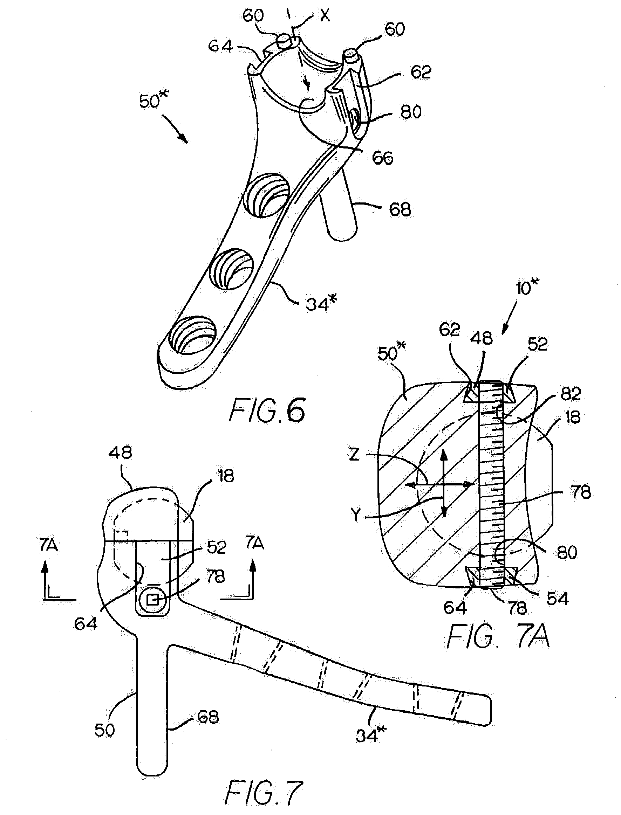

Figure 6 shows a base 50* which is very similar to the base 50 of Figure 3, except the cup 70 has been removed and a pin 68 has been added.

The base 50* includes a mounting leg 34* for mounting the socket on the radius bone. The base 50* has an inner surface 66, which defines a partial sphere. It also has an outer surface that defines first and second grooves 62, 64, which are parallel and opposite to each other and which extend in a first direction "X". When the cap 48 and base 50* are assembled together, with the first and second legs 52, 54 received in the first and second grooves 62, 64, the inner surfaces 56, 66 together form a socket, which is sized and shaped to receive the spherical ball 18 of the prosthesis 10* and to support the ball 18 for rotation. As in the prior art arrangement of Figure 2, the socket forms a smooth, nearly continuous inner surface to provide for smooth rotation of the ball 18. The assembled base 50* and cap 48 function in essentially the same manner as the base 20 and cap 40 of Figure 2, capturing the ball 18 and allowing the ball 18 to rotate.

The grooves 62, 64 of the base 50* define internally threaded, through openings 80, 82 (only one shown in Figure 6 but both visible in Figure 7A), which have an axis extending in a direction that is cross-wise to the first direction "X". These threaded openings 80, 82 receive the externally threaded fastener pin 78 (See Figures 3 and 4) as described in more detail later.

It should be noted that the first direction "X" of Figure 6 is the same as the first direction "X" of Figure 5, and is represented as well in Figure 3 as being the direction in which the legs 52, 54 slide into the grooves 62, 64 to assemble the cap 48 and base 50* together. Of course, to disassemble the cap 48 from the base 50*, the cap 48 would be moved in the opposite direction relative to the base 50* in order to slide the legs 52, 54 out of the grooves 62, 64.

The appendage 68 shown extending from the back of the base 50* is inserted into a hole drilled in the radius bone 22 to help fix the position of the base 50* relative to the bone.

The cap 48 also defines two recesses 58 (only one is visible in Figure 5) sized and located so as to receive mating projections 60 (See Figure 6) on the base 50*.

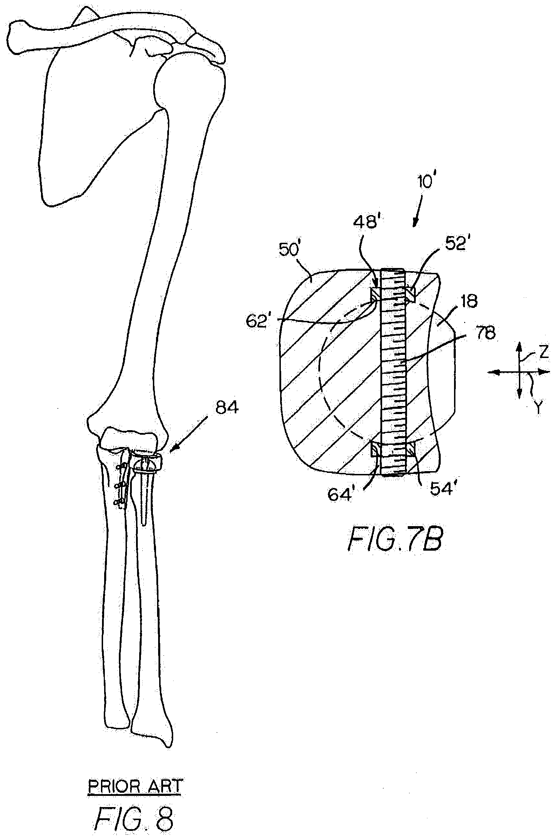

Figure 7 shows the assembly of the cap 48, the base 50*, the ball 18, and the fastener pin 78. The stem 16, which is assembled to the ball 18, is omitted for simplicity. To assemble the prosthesis 10*, the ball 18 is placed between the partial spherical cavities 56, 66, of the cap 48 and base 50*, and then the cap 48 and base 50* are brought together in the first direction "X" (See Figure 3), wherein the legs 52, 54 of the cap 48 slide into the grooves 62, 64 respectively of the base 50* until the projections 60 are received in the recesses 58 and the through openings 74, 76 of the cap 48 are aligned with the corresponding through openings 80, 82 in the base 50*. The fastener pin 78 is then threaded through all four aligned openings 74, 80, 76, 82, with the axis of the fastener pin 78 extending cross-wise to the first direction "X". Note that the fastener pin 78 may be inserted from either side of the prosthesis 10* which allows the same prosthesis to be used for either a "left hand" application or a "right hand" application.

A closer look at Figure 7A shows that the sides of the legs 52, 54 taper outwardly and that the sides of the grooves 62, 64, which receive the legs 52, 54, taper inwardly with a mating taper. When the legs 52, 54 are received in the grooves 62, 64, as shown in Figure 7A, the tapered surfaces of the grooves 62, 64 partially overlie and contact the mating outer surfaces of the legs 52, 54, which prevents the legs 52, 54 from moving outwardly, in the "Y" direction, away from their respective grooves 62, 64. As soon as the legs 52, 54 are received in the grooves 62, 64, the legs 52, 54 are prevented from moving in any direction other than the X direction relative to the grooves. And once the fastener pin 78 is threaded into the prosthesis 10*, the legs 52, 54 also are prevented from moving in the first direction "X", thereby fixing the cap 48 relative to the base 50* in every direction.

Any force acting to separate the cap 48 from the base 50* in the "X" direction (the direction to slide the legs 52, 54 out of the grooves 60, 62) applies shear forces to the fastener pin 78. This does not tend to unthread the fastener pin 78 and does not rely on the engagement of the threads to hold the parts together. Instead, it relies on the body of the fastener pin 78 itself, since, as long as the pin 78 is in place, one would have to shear the pin (cut it entirely through) in order to slide the legs 52, 54 out of their respective grooves 60, 62.

Figure 7B shows a second embodiment of the prosthesis 10'. In this embodiment, the legs 52', 54' of the cap 48' are received in grooves 62', 64' formed on the inner surface of the base. In this case, the space between the legs 52', 54' is less than in the previous embodiment, and the inner surfaces of the legs 52', 54' have a partial spherical shape and become part of the socket that is in contact with the ball 18. In this case, the ball 18 prevents the legs 52', 54' from moving inwardly, and the inner surface of the cap base 50' that contacts the outer surface of the legs 52', 54' prevents the legs 52', 54' from moving apart outwardly, in the "Y" direction. The edges of the grooves 62', 64' prevent the legs from moving in the "Z" direction.

It should be noted that either of the members may be considered to be the base, with the other member being the cap, so the grooves may be placed on either member, and the legs would then be placed in the other member. In addition, it would be possible to put both a groove and a leg on the cap and a corresponding mating leg and groove on the base member.

It should also be noted that, whether the legs 52, 54 are placed on the outside of the base 50 or the cap 48, or placed on the inside of the base 50 or the cap 48, the socket has a smooth, nearly continuous surface to provide for smooth rotation of the ball in the socket. Also, in the embodiment 10* the through openings 74, 76 on the legs 52, 54 are located adjacent the mounting leg 34*, with the remainder of the outer surfaces of the base 50 and the cap 48 being smooth and nearly continuous, in order to minimize the opportunity for the prosthesis to catch on or interfere with ligaments when the prosthesis is installed. In contrast, the prior art design, shown in Figure 2, has fasteners 44 on the outer surface of the cap 40, with recesses that could catch on ligaments.

While the present mounting arrangements for a ball-and-socket joint have been described with respect to a wrist joint, they also could be used for other ball-and-socket joints, such as the elbow and the base of the thumb. Examples of these are described briefly below.

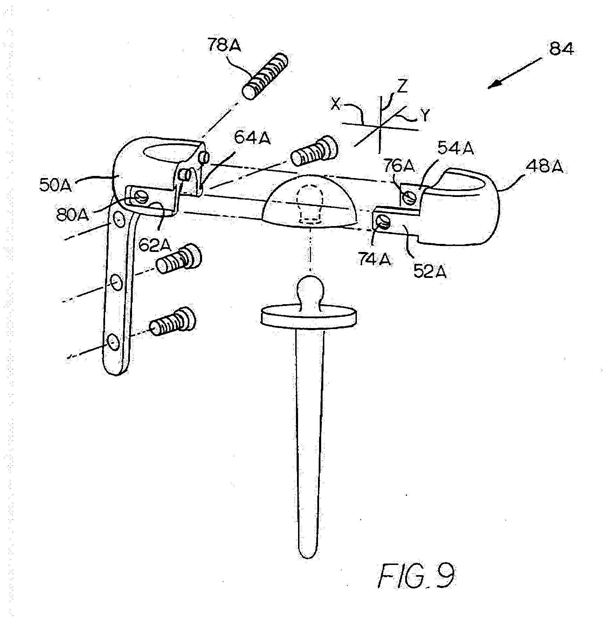

Figure 8 shows a prior art elbow prosthesis 84, as described in U.S. Patent 8,048,162, Lateral Elbow Prosthesis - Proximal Radioulnar Joint, dated November 1, 2011. Figure 9 is an exploded view of an elbow prosthesis that is similar to the prosthesis of Figure 8 but with an improved socket 84* that has a more secure connection, similar to the wrist connection described earlier. It may be seen that the cap 48A includes legs 52A, 54A which extend in the "X" direction. The base 50A defines grooves 62A, 64A which receive the legs 52A, 54A of the cap 48A. The fastener pin 78A extends in a cross-wise direction through aligned openings 80A in the base 50A, and 74A, 76A in the cap 48A to releasably secure the cap 48A to the base 50A, with the fastener pin 78A under shear loading as forces act to try to separate the cap 48A from the base 50A in the "X" direction. As with the wrist prostheses described earlier, the grooves 62A, 64A restrict the legs 52A, 54A to movement in the X direction. The legs 52A, 54A and grooves 62A, 64A have mating tapers that are the same as those shown in Figure 7A for the wrist.

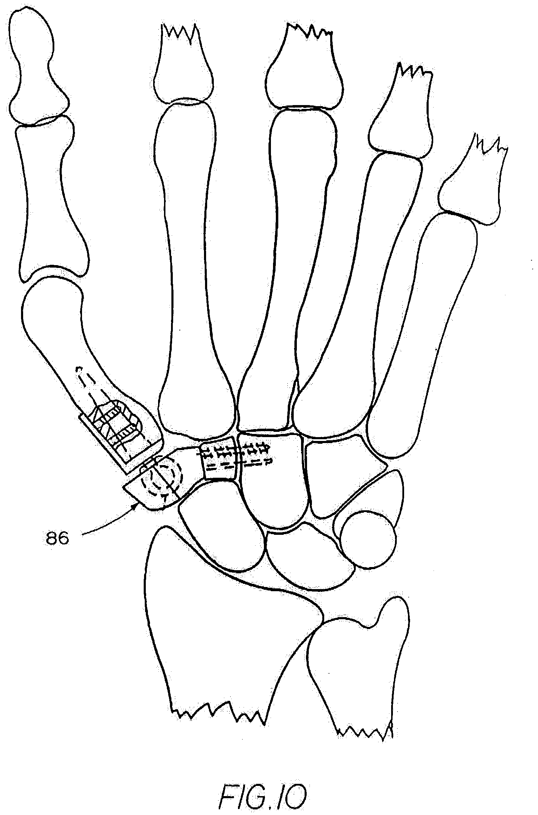

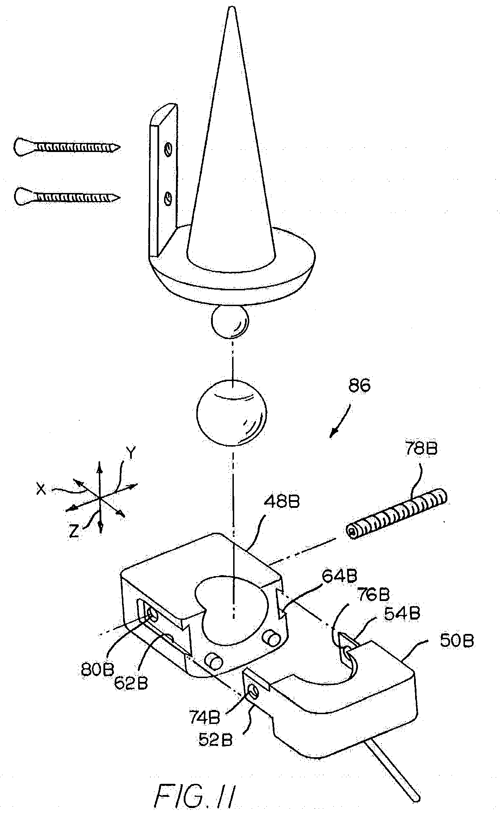

Figures 10 and 11 show a prosthesis 86 for the base of the thumb which uses a similar mounting arrangement for the cap and base. Figure 11 is an exploded view of the thumb prosthesis of Figure 10. It may be seen that the base 50B includes legs 52B, 54B which extend in the "X" direction. The cap 48B defines grooves 62B, 64B which receive the legs 52B, 54B of the base 50. The fastener pin 78B extends in a cross-wise direction through aligned openings 80B in the cap 48B, and 74B, 76B in the base 50B to releasably secure the cap 48B to the base 50B, with the fastener pin 78B under shear loading as forces act to separate the cap 48B from the base 50B in the "X" direction. The legs 52B, 54B are received in and engage the grooves 62B, 64B to restrict movement of the cap 48B relative to the base 50B to movement in the X direction. The legs 52B, 54B have tapered edges that are mated to the tapered edges of the grooves 62B, 64B and function in the same way as the mated tapered edges described earlier.

It will be obvious to those skilled in the art that modifications may be made to the embodiments described above without departing from the scope of the present invention as defined by the claims.

## Claims

1. A prosthesis (10) for replacing a mammalian joint, comprising:
a first body (16), including a ball (18) mounted on a shaft;
a second body (12), including a base (50) and a cap (48) which secures to the base, said base and cap each having an inner surface (56, 66) and an outer surface, with the inner surfaces of the base and cap together defining a socket which receives and traps the ball and supports the ball for rotation relative to the second body;
**characterized in that** one of said inner and outer surfaces of said base defines a first groove (62), and said cap has a first leg (52) projecting in a first direction and being received in said first groove by sliding into said first groove in said first direction;
and further in comprising a second leg (54) and a second groove (64) lying parallel to and directly opposite said first leg and said first groove,
wherein said first leg defines a first leg opening (74) having an axis extending in a direction that is cross-wise to the first direction, said second leg defines a second leg opening (76) coaxial with the first leg opening, and said base defines a base opening which is aligned with said first leg opening when the base and cap are assembled together; and
a pin (78) received in said first leg opening, said second leg opening, and said base opening and extending in the cross-wise direction to secure the cap and base together so that an attempt to slide the cap and base apart from each other in the first direction applies shear forces to the pin; and
means for preventing the first leg from moving outwardly away from the first groove in the cross-wise direction when the first leg is received in the first groove;
wherein said means for preventing the first leg from moving outwardly away from the first groove includes at least a portion of said base contacting the outer surface of the first leg.

2. A prosthesis for replacing a mammalian joint as recited in claim 1, wherein said first groove lies on the outer surface of said base and has edges that overlie the outer surface of the first leg when the first leg is inserted into the first groove.

3. A prosthesis for replacing a mammalian joint as recited in claim 1 or 2, wherein said means includes said first groove lying on the inner surface of said base.

4. A prosthesis for replacing a mammalian joint as recited in claim 1, 2 or 3 wherein at least one of said first leg opening and said base opening is a threaded opening, and said pin has threads which mate with corresponding threads in said threaded opening.

5. A prosthesis for replacing a mammalian joint as recited in claim 4, wherein said pin can be installed both from the first leg side of the prosthesis and from the second leg side of the prosthesis.

6. A prosthesis for replacing a mammalian joint as recited in claim 4 or 5, wherein the socket has a smooth, nearly continuous surface to provide for smooth rotation of the ball in the socket.

7. A prosthesis for replacing a mammalian joint as recited in claim 4, 5 or 6, wherein the base defines a mounting leg (34) projecting away from the socket for mounting the socket on a bone, the first and second leg openings are adjacent to said mounting leg, and the remainder of the outer surfaces of said base and cap are smooth and nearly continuous, in order to minimize the opportunity for the prosthesis to catch on or interfere with ligaments when the prosthesis is installed.

8. A prosthesis for replacing a mammalian joint as recited in any preceding claim, wherein each of said first and second grooves lies on the inner surface of the respective cap or base member on which the respective groove is located.

9. A prosthesis for replacing a mammalian joint as recited in any preceding claim, wherein each of said first and second grooves lies on the outer surface of the respective cap or base member on which the respective groove is located, and wherein each of said first and second grooves has edges that overlie the outer surface of the respective leg that is received in the respective groove when the legs are inserted into the grooves.

10. A prosthesis for replacing a mammalian joint as recited in any preceding claim, wherein said prosthesis is a replacement for the joint at the base of the thumb.

11. A prosthesis for replacing a mammalian joint as recited in any one of claims 1 to 9, wherein said prosthesis is a replacement for the wrist joint.

12. A prosthesis for replacing a mammalian joint as recited in any one of claims 1 to 9, wherein said prosthesis is a replacement for the elbow joint.

## Patentansprüche

1. Prothese (10) zum Ersetzen eines Säugetiergelenks, die Folgendes umfasst:
einen ersten Körper (16), der eine Kugel (18) beinhaltet, die an einem Schaft montiert ist;
einen zweiten Körper (12), der eine Basis (50) und eine Kappe (48), die an der Basis befestigt ist, beinhaltet, wobei die Basis und die Kappe jeweils eine Innenfläche (56, 66) und eine Außenfläche aufweisen, wobei die Innenflächen der Basis und der Kappe zusammen eine Buchse definieren, die die Kugel aufnimmt und einschließt und die Kugel zum Drehen relativ zum zweiten Körper stützt;
**dadurch gekennzeichnet, dass**
eine der Innen- und der Außenfläche der Basis eine erste Rille (62) definiert und die Kappe ein erstes Bein (52) aufweist, das durch Gleiten in die erste Rille in eine erste Richtung in die erste Richtung vorsteht und in die erste Rille aufgenommen wird;
und ferner dadurch, dass sie ein zweites Bein (54) und eine zweite Rille (64) umfasst, die parallel zum ersten Bein und zur ersten Rille und diesen direkt gegenüberliegen,
wobei das erste Bein eine erste Beinöffnung (74) mit einer Achse, die sich in eine Richtung erstreckt, die quer zur ersten Richtung verläuft, definiert und das zweite Bein eine zweite Beinöffnung (76) koaxial zur ersten Beinöffnung definiert und die Basis eine Basisöffnung definiert, die auf die erste Beinöffnung ausgerichtet ist, wenn die Basis und die Kappe zusammenmontiert sind; und
einen Stift (78), der in die erste Beinöffnung, die zweite Beinöffnung und die Basisöffnung aufgenommen ist und sich in die Querrichtung erstreckt, um die Kappe und die Basis aneinander zu sichern, derart, dass bei einem Versuch, die Kappe und die Basis durch Gleiten in die erste Richtung voneinander zu trennen, Scherkräfte auf den Stift aufgebracht werden; und
ein Mittel zum Verhindern, dass sich das erste Bein von der ersten Rille weg nach außen in die Querrichtung bewegt, wenn das erste Bein in die erste Rille aufgenommen wird;
wobei das Mittel zum Verhindern, dass sich das erste Bein von der ersten Rille weg nach außen bewegt, beinhaltet, dass mindestens ein Abschnitt der Basis die Außenfläche des ersten Beins berührt.

2. Prothese zum Ersetzen eines Säugetiergelenks nach Anspruch 1, wobei die erste Rille auf der Außenfläche der Basis liegt und Ränder aufweist, die auf der Außenfläche des ersten Beins aufliegen, wenn das erste Bein in die erste Rille eingesteckt wird.

3. Prothese zum Ersetzen eines Säugetiergelenks nach Anspruch 1 oder 2, wobei das Mittel beinhaltet, dass die erste Rille auf der Innenfläche der Basis liegt.

4. Prothese zum Ersetzen eines Säugetiergelenks nach Anspruch 1, 2 oder 3, wobei mindestens eine der ersten Beinöffnung und der Basisöffnung eine Gewindeöffnung ist und der Stift ein Gewinde aufweist, das in ein entsprechendes Gewinde in der Gewindeöffnung eingreift.

5. Prothese zum Ersetzen eines Säugetiergelenks nach Anspruch 4, wobei der Stift sowohl von der Seite des ersten Beins der Prothese als auch von der Seite des zweiten Beins der Prothese installiert werden kann.

6. Prothese zum Ersetzen eines Säugetiergelenks nach Anspruch 4 oder 5, wobei die Buchse eine glatte, nahezu durchgehende Fläche aufweist, um eine glatte Drehung der Kugel in der Buchse bereitzustellen.

7. Prothese zum Ersetzen eines Säugetiergelenks nach Anspruch 4, 5 oder 6, wobei die Basis ein Montagebein (34) definiert, das zum Montieren der Buchse an einem Knochen von der Buchse vorsteht, die erste und die zweite Beinöffnung dem Montagebein benachbart sind und der Rest der Außenflächen der Basis und der Kappe glatt und nahezu durchgehend sind, um die Gelegenheit zu minimieren, dass sich die Prothese an Bändern verhakt oder diese beeinträchtigt, wenn die Prothese installiert wird.

8. Prothese zum Ersetzen eines Säugetiergelenks nach einem der vorhergehenden Ansprüche, wobei jede der ersten und der zweiten Rille auf der Innenfläche der jeweiligen Kappe oder des jeweiligen Basiselements, auf der bzw. dem sich die jeweilige Rille befindet, liegt.

9. Prothese zum Ersetzen eines Säugetiergelenks nach einem der vorhergehenden Ansprüche, wobei jede der ersten und der zweiten Rille auf der Außenfläche der jeweiligen Kappe oder des jeweiligen Basiselements, auf der bzw. dem sich die jeweilige Rille befindet, liegt und wobei jede der ersten und der zweiten Rille Ränder aufweist, die auf der Außenfläche des jeweiligen Beins, das in die jeweilige Rille aufgenommen ist, aufliegen, wenn die Beine in die Rillen eingesetzt werden.

10. Prothese zum Ersetzen eines Säugetiergelenks nach einem der vorhergehenden Ansprüche, wobei die Prothese ein Ersatz für das Gelenk an der Basis des Daumens ist.

11. Prothese zum Ersetzen eines Säugetiergelenks nach einem der Ansprüche 1 bis 9, wobei die Prothese ein Ersatz für das Handgelenk ist.

12. Prothese zum Ersetzen eines Säugetiergelenks nach einem der Ansprüche 1 bis 9, wobei die Prothese ein Ersatz für das Ellenbogengelenk ist.

## Revendications

1. Prothèse (10) de remplacement d'une articulation de mammifère, comprenant :
un premier corps (16), comportant une bille (18) montée sur une tige ;
un deuxième corps (12), comportant une base (50) et une coiffe (48) qui s'arrime à la base, ladite base et ladite coiffe ayant chacune une surface interne (56, 66) et une surface externe, les surfaces internes de la base et de la coiffe définissant ensemble une emboîture qui reçoit et piège la bille et support la bille en rotation par rapport au deuxième corps ;
**caractérisée en ce que** :
l'une desdites surfaces interne et externe de ladite base définit une première rainure (62), et ladite coiffe a une première patte (52) en saillie dans une première direction et reçue dans ladite première rainure par coulissement dans ladite première rainure dans ladite première direction ;
et en outre **en ce qu'**elle comprend une deuxième patte (54) et une deuxième rainure (64) disposées parallèles et directement opposées à ladite première patte et à ladite première rainure ;
dans laquelle ladite première patte définit une première ouverture de patte (74) ayant un axe s'étendant dans une direction qui est transversale à la première direction, ladite deuxième patte définit une deuxième ouverture de patte (76) coaxiale à la première ouverture de patte, et ladite base définit une ouverture de base qui est alignée avec ladite première ouverture de patte lorsque la base et la coiffe sont assemblées ensemble ; et
une broche (78) reçue dans ladite première ouverture de patte, ladite deuxième ouverture de patte, et ladite ouverture de base et s'étendant dans la direction transversale pour arrimer la coiffe et la base ensemble de sorte qu'une tentative de séparation de la coiffe et de la base l'une de l'autre par coulissement dans la première direction applique des forces de cisaillement à la broche ; et
un moyen destiné à empêcher la première patte de se déplacer vers l'extérieur en éloignement de la première rainure dans la direction transversale lorsque la première patte est reçue dans la première rainure ;
dans laquelle ledit moyen destiné à empêcher la première patte de se déplacer vers l'extérieur en éloignement de la première rainure comporte au moins une partie de ladite base en contact avec la surface externe de la première patte.

2. Prothèse pour le remplacement d'une articulation de mammifère selon la revendication 1, dans laquelle ladite première rainure est disposée sur la surface externe de ladite base et a des bords qui recouvrent la surface externe de la première patte lorsque la première patte est insérée dans la première rainure.

3. Prothèse pour le remplacement d'une articulation de mammifère selon la revendication 1 ou 2, dans laquelle ledit moyen comporte la première rainure disposée sur la surface interne de ladite base.

4. Prothèse pour le remplacement d'une articulation de mammifère selon la revendication 1, 2 ou 3, dans laquelle au moins l'une de ladite première ouverture de patte et de ladite ouverture de base est une ouverture filetée, et ladite broche a des filets qui s'accouplent avec des filets correspondants dans ladite ouverture filetée.

5. Prothèse pour le remplacement d'une articulation de mammifère selon la revendication 4, dans laquelle ladite broche peut être installée à la fois depuis le côté de première patte de la prothèse et depuis le côté de deuxième patte de la prothèse.

6. Prothèse pour le remplacement d'une articulation de mammifère selon la revendication 4 ou 5, dans laquelle l'emboîture a une surface lisse presque continue pour assurer une rotation lisse de la bille dans l'emboîture.

7. Prothèse pour le remplacement d'une articulation de mammifère selon la revendication 4, 5 ou 6, dans laquelle la base définit une patte de montage (34) en saillie en éloignement de l'emboîture pour monter l'emboîture sur un os, les première et deuxième ouvertures de patte sont adjacentes à ladite patte de montage, et le reste des surfaces externes de ladite base et de ladite coiffe sont lisses et presque continues, afin de minimiser la possibilité pour la prothèse d'accrocher les ligaments ou d'interférer avec eux lorsque la prothèse est installée.

8. Prothèse pour le remplacement d'une articulation de mammifère selon l'une quelconque des revendications précédentes, dans laquelle chacune desdites première et deuxième rainures est disposée sur la surface interne de l'élément de coiffe ou de base respectif sur lequel est située la rainure respective.

9. Prothèse pour le remplacement d'une articulation de mammifère selon l'une quelconque des revendications précédentes, dans laquelle chacune desdites première et deuxième rainures est disposée sur la surface interne de l'élément de coiffe ou de base respectif sur lequel est située la rainure respective, et dans laquelle chacune desdites première et deuxième rainures a des bords qui recouvrent la surface externe de la patte respective qui est reçue dans la rainure respective lorsque les pattes sont insérées dans les rainures.

10. Prothèse pour le remplacement d'une articulation de mammifère selon l'une quelconque des revendications précédentes, ladite prothèse étant un remplacement pour l'articulation à la base du pouce.

11. Prothèse pour le remplacement d'une articulation de mammifère selon l'une quelconque des revendications 1 à 9, ladite prothèse étant un remplacement pour l'articulation du poignet.

12. Prothèse pour le remplacement d'une articulation de mammifère selon l'une quelconque des revendications 1 à 9, ladite prothèse étant un remplacement pour l'articulation du coude.
